# EUROPEAN PATENT APPLICATION

(11) **EP 4 520 379 A1**
(43) Date of publication of application: **12.03.2025**
(21) Application number: 24198154.7
(22) Date of filing: 03.09.2024
(51) Int. Cl.: A61M 25/02

(54) **ANCHORING SYSTEM FOR CATHETERS**

(30) Priority: 08.09.2023 US 202363581578 P; 22.11.2023 US 202363602312 P
(71) Applicant: Bedal, 3590 Diepenbeek (BE)
(72) Inventor: BIERMAN, Steven F., BE 3590 Diepenbeek (BE); CALLAERTS, Simon, BE 3590 Diepenbeek (BE); LACERTOSA, Luca, BE 3590 Diepenbeek (BE); VAN DAMME, Alexander, BE 3590 Diepenbeek (BE)
(74) Representative: Kirklies, Michael Dieter

(57) **Abstract**

An anchoring system (10) includes a simply structured suture-like device which permits a catheter to be easily attached to the patient without the use of needles and without actually suturing anything to the patient. The anchoring device (10) includes two filaments (20) coupled to a base (18) and two corresponding receptacles (23) with slots. The filaments (20) include protuberances which cooperate with apertures of the receptacles to permit easy insertion and removal of a midportion of the filaments into a side slot of the receptacles (23) in a longitudinal direction. The receptacles (23) inhibit retraction of the filament (20) in a lateral direction from the receptacles. The base (18) includes an adhesive bottom surface which can be attached to the patient's skin. The base (18) can further include an adhesive top surface or platform configured to adhere to a bottom surface of the catheter.

## Description

### CROSS-REFERENCE TO RELATED APPLICATIONS

This application claims benefit under 35 U.S.C. 119(e) to U.S. Provisional Patent Application No. 63/581,578, filed September 8, 2023 and U.S. Provisional Patent Application No. 63/602,312, filed November 22, 2023, the entire disclosures of which are hereby incorporated by reference herein in their entireties. Any and all priority claims identified in the Application Data Sheet, or any corrections thereto, are hereby incorporated by reference under 37 CFR 1.57.

### BACKGROUND

### Field

The present disclosure relates in general to a universal anchoring system for securing a medical article to a patient and, in particular, to a universal anchoring system for securing a catheter to a patient to inhibit movement or migration of the catheter relative to the patient.

### DESCRIPTION OF RELATED ART

It is common in the treatment of patients to utilize catheters to introduce fluids and medications directly into the patient or to withdraw fluids from the patient. An example of a typical catheter is a central venous catheter, or CVC, which is used to introduce fluids through a central vein.

In most cases, the catheter remains in place for many days. In order to secure the catheter in position at the insertion site, a healthcare worker often secures the catheter to the patient using tape. That is, the healthcare worker commonly places long pieces of tape across the portion of the catheter near the insertion site in a crisscross pattern to secure the catheter to the patient's skin. This securement inhibits disconnection between the catheter and the insertion site. Tape, however, often collects dirt and other contaminates. Normal protocol therefore requires periodic (e.g., daily) tape changes to inhibit bacteria and germ growth at the securement site.

A variety of catheter securement devices have been developed to obviate the need for frequent application of tape to secure a catheter to a patient. Many of these securement devices, however, suffer from one or more of the following disadvantages: are time consuming and inconvenient to secure; have multiple parts, which can be dropped and become none sterile; and require removal instruments (e.g., hemostat or scissors) to disengage the catheter from the securement device.

### SUMMARY

The present disclosure is directed to an anchoring system for securing a catheter to the body of a patient. The system comprises a catheter comprising a wing having at least one suture hole formed therein and an anchoring apparatus comprising an adhesive member for secure attachment to the body of the patient, a base mounted on the adhesive member and having at least one receptacle, the at least one receptacle forming a channel through a portion of the base, the channel having a central axis and a slot through a side of the channel, and at least one filament having a proximal end, a distal end, and a midportion therebetween, the proximal end being coupled to the base, the distal end being configured to be inserted through the suture hole of the wing, the midportion being sized and shaped to be inserted into and subsequently removed from the channel via the slot, wherein the at least one filament is inhibited from being withdrawn from the channel in a direction along the central axis towards the wing when the midportion of the at least one filament is in the channel.

Additional aspects further comprise wherein the central axis is parallel to a lateral direction.

Additional aspects further comprise wherein the at least one filament comprises two filaments, and wherein the at least one receptacle comprises two receptacles.

Additional aspects further comprise wherein the at least one filament comprises a platform configured to lock to the base.

Additional aspects further comprise wherein the at least one filament comprises a protuberance configured to abut a surface of the receptacle when the midportion of the at least one filament is in the channel.

Additional aspects further comprise wherein the base further comprises an adhesive platform configured to contact at least a portion of the catheter.

Additional aspects further comprise wherein the channel has a C-shape.

Additional aspects further comprise wherein the slot faces in a longitudinal direction towards an insertion site.

Additional aspects further comprise wherein the base comprises at least one sidewall, and wherein the at least one filament extends in a lateral direction from the at least one sidewall.

Additional aspects further comprise wherein the at least one sidewall is a vertical member or surface.

The present disclosure is further directed to an anchoring device for securing a catheter to the body of a patient. The catheter has a wing with at least one suture hole formed therein. The anchoring device comprises an adhesive member for secure attachment to the body of the patient, a base mounted on the adhesive member and having at least one receptacle, the at least one receptacle forming a channel through a portion of the base, the channel having a central axis and a slot through a side of the channel, and at least one filament having a proximal end, a distal end, and a midportion therebetween, the proximal end being coupled to the base, the distal end being configured to be inserted through the suture hole of the wing, the midportion being sized and shaped to be inserted into and subsequently removed from the channel via the slot. The at least one filament is inhibited from being withdrawn from the channel in a direction along the central axis towards the wing when the midportion of the at least one filament is in the channel.

Additional aspects further comprise wherein the at least one receptacle comprises two receptacles and the at least one filament comprises two filaments, and wherein the midportions of the two filaments extend along a lateral direction when secured in the two receptacles.

Additional aspects further comprise wherein the midportions of the two filaments extend in opposite lateral directions away from each other when secured in the two receptacles.

Additional aspects further comprise wherein the at least one filament comprises a platform configured to lock to the base.

Additional aspects further comprise wherein the at least one filament comprises a protuberance configured to abut a surface of the receptacle when the midportion of the at least one filament is in the channel.

Additional aspects further comprise wherein the base further comprises an adhesive platform configured to contact at least a portion of the catheter.

Additional aspects further comprise wherein the channel has a C-shape.

Additional aspects further comprise wherein the slot faces in a longitudinal direction towards an insertion site.

Additional aspects further comprise wherein the base comprises at least one sidewall, and wherein the at least one filament extends in a lateral direction from the at least one sidewall.

Additional aspects further comprise wherein the at least one sidewall is a vertical member or surface.

The present disclosure is further directed to a method for securing a catheter to a body of a patient. The catheter has a wing with at least one suture hole formed therein. The method comprises aligning the at least one suture hole with an end of a filament, the filament being coupled to a base and extending in a transversal direction from the base, the base comprising at least one receptacle, threading the end of the filament through the at least one suture hole, wrapping the filament around the wing, and pulling the filament in a lateral direction towards the base to secure a midportion of the filament in the at least receptacle so as to retain the catheter.

Additional aspects further comprise wherein pulling the filament comprises pulling the filament taut so as to stretch a length of the filament.

Additional aspects further comprise wherein the at least one receptacle comprises two receptacles and the filament comprises two filaments, and wherein the midportions of the two filaments extend along a lateral direction when secured in the two receptacles.

Additional aspects further comprise wherein the midportions of the two filaments extend in opposite lateral directions away from each other when secured in the two receptacles.

Additional aspects further comprise hand manipulating the filament to disengage the filament from the at least one receptacles.

### BRIEF DESCRIPTION OF THE DRAWINGS

Various embodiments are depicted in the accompanying drawings for illustrative purposes and should in no way be interpreted as limiting the scope of the embodiments. In addition, various features of different disclosed embodiments can be combined to form additional embodiments, which are part of this disclosure.
FIG. 1 is a perspective view of an anchoring system in accordance with a preferred embodiment of the present invention including at least one filament;
FIG. 2 is a top plan view of the anchoring system from FIG. 1;
FIG. 3 is a left side view of the anchoring system from FIG. 2;
FIG. 4 is a right side view of the anchoring system from FIG. 2;
FIG. 5 is a back view of the anchoring system from FIG. 2;
FIG. 6 is a front view of the anchoring system from FIG. 2;
FIG. 7 is a cross sectional view of the anchoring system of FIG. 2 taken along line A-A;
FIG. 8 is a bottom view of the anchoring system from FIG. 2;
FIG. 9 is an exploded view of the anchoring system from FIG. 1;
FIG. 10 is a top plan view of the retainer of the anchoring system from FIG. 1 with the at least one filament assembled to the base;
FIG. 11 is a left side view of the retainer from FIG. 10;
FIG. 12 is a right side view of the retainer from FIG. 10;
FIG. 13 is a back view of the retainer from FIG. 10;
FIG. 14 is a front view of the retainer from FIG. 10;
FIG. 15 is a bottom view of the retainer from FIG. 10;
FIG. 16 is a cross sectional view of the retainer of FIG. 10 taken along line B-B showing the at least one filament fully assembled to the base;
FIG. 17 is an enlarged view from FIG. 16 taken in view C with the filament pulled through the base and the shoulder of the filament locked in the base;
FIG. 18 is an exploded view of the retainer from FIG. 10 showing the at least one filament prior to being assembled to the base;
FIG. 19 is a top plan view of the retainer of the anchoring system from FIG. 1 with the at least one filament partially assembled to the base;
FIG. 20 is a cross sectional view of the retainer of FIG. 19 taken along line D-D showing the at least one filament partially assembled to the base;
FIG. 21 is an enlarged view from FIG. 20 taken in view E with the at least one filament pulled through the base but before the shoulder of the filament is locked in the base;
FIG. 22 includes views of a central venous catheter (CVC) partially secured to the anchoring system but before the filaments are locked to the base;
FIG. 23 is a back view of the CVC and anchoring system from FIG. 22;
FIG. 24 is a front view of the CVC and anchoring system from FIG. 22;
FIG. 25 is a cross sectional view of the CVC and anchoring system from FIG. 22 taken along line F-F;
FIG. 26 is a top plan view of the CVC and anchoring system from FIG. 22 after the filaments are locked to the base; and
FIG. 27 is a top plan view of the central venous catheter (CVC) from FIG. 26.
FIG. 28 is a perspective view of another preferred embodiment of an anchoring system in accordance with the present invention including at least one filament;
FIG. 29 is a top plan view of the anchoring system from FIG. 28;
FIG. 30 is a front view of the anchoring system from FIG. 28;
FIG. 31 is a side view of the anchoring system taken along lines 31-31 in FIG. 30;
FIG. 32 is a perspective view of apertures in wings of a central venous catheter (CVC) aligned for engagement with the filaments of the anchoring system of FIG. 28 during securement;
FIG. 33 is similar to FIG. 32 except the filaments have been threaded through the corresponding apertures in the CVC and then fed through the corresponding receptacles to lock the filaments to the bases of the anchoring system;
FIG. 34 is a top plan view of the CVC secured to the anchoring system of FIG. 28;
FIG. 35 is a front view of the CVC and anchoring system taken along lines 35-35 in FIG. 34; and
FIG. 36 is a side view of the CVC and anchoring system taken along lines 36-36 in FIG. 34.

### DETAILED DESCRIPTION

The present description will be directed in particular to elements forming part of, or cooperating more directly with, apparatus and methods in accordance with the present invention. It is to be understood that elements not specifically shown or described may take various forms well known to those skilled in the art.

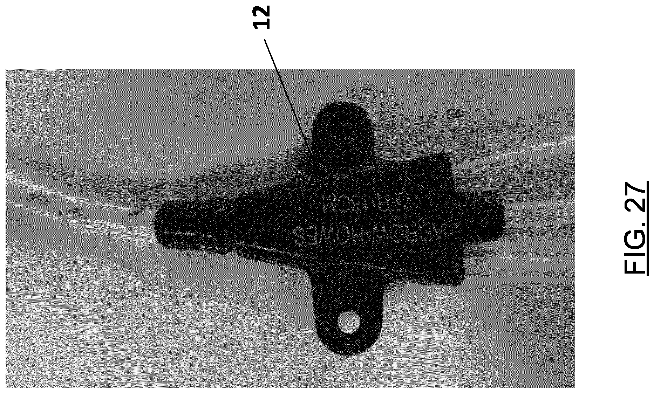

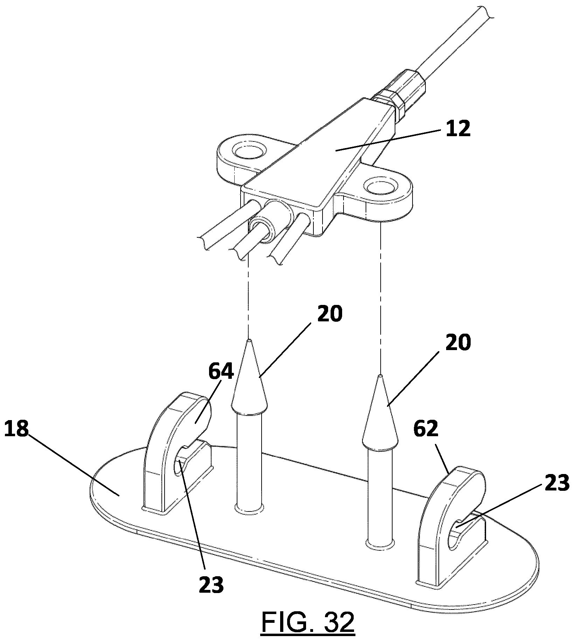

The present embodiment of the catheter anchoring system, which is generally designated by reference numeral 10, is disclosed in the context of use with an exemplary CVC (as shown in FIGS. 27 and 32 and designated by reference numeral 12). The principles of the present invention, however, are not limited to catheters. Instead, it will be understood by one of ordinary skill in this art, in view of the present disclosure, that the anchoring system and/or retainer disclosed herein also can be successfully utilized in connection with other types of medical articles, including, but not limited to, other types of catheters, fluid drainage and delivery tubes and electrical wires. For example, but without limitation, the retainer disclosed herein also can be configured to receive and secure peripheral catheters, peripherally inserted central catheters, hemodialysis catheters, surgical drainage tubes, feeding tubes, chest tubes, nasogastric tubes, scopes, as well as electrical wires or cables connected to external or implanted electronic devices or sensors. One skilled in the art may also find additional applications for the devices and systems disclosed herein. Thus, the illustration and description of the anchoring system in connection with a CVC is merely exemplary of one possible application of the anchoring system.

Each of the embodiments described herein employs some basic concepts characteristic of the present anchoring system, namely releasable engagement of the catheter to the patient. The releasable engagement is achieved by cooperation among a base, at least one filament, and at least one receptacle. This cooperation allows the catheter to be disconnected from the anchoring system and from the patient, for any of a variety of known purposes. For instance, the healthcare worker may want to remove the catheter from the anchoring system to ease disconnection of the catheter from the insertion point or to clean the patient before reinstallation. The disengagement of the catheter from the anchoring system, however, can be accomplished without removing the anchoring system from the patient. The disengagement of the catheter from the anchoring system can be accomplished without the need for removal instruments (e.g., hemostat or scissors). The healthcare worker can simply manipulate the filaments by hand to disengage the filaments from the receptacles. Once disengaged, the healthcare worker can remove the catheter from the anchoring system and clean the catheter if desired. Once cleaned, the healthcare worker can secure the same catheter to the same anchoring system by simple hand manipulation of the original filaments. In this way, the catheter can be secured, unsecured and cleaned and secured again repeatedly without replacement or use of a removal instrument. The anchoring system allows for reversible securement.

Common to each the described embodiments, the present anchoring system inhibits axial movement of the catheter with respect to the anchoring system, and hence, with respect to the insertion site on the patient. Transverse and lateral movement is generally arrested by the holding effect provided by the base, as well as by the cooperative interaction between at least one filament and an opening on the catheter and/or catheter fitting. That is, the base supports the catheter while the filament(s) extends through a corresponding suture or mount opening(s) formed on the catheter and/or catheter fitting. Longitudinal movement is similarly arrested by the interaction between the filament(s) and the opening(s).

In certain embodiments, at least a portion of the filament extends in a lateral direction towards the center of the catheter to form a landing pad for the wing of the catheter. In certain embodiments, the filament extends in the lateral direction from a sidewall of the base. For example, in certain embodiments, the sidewall is a vertical member or surface of the base that extends in the transverse direction. For example, in certain embodiments, the filament emerges horizontally from the vertical member or surface of the base. Such an embodiment is in contrast to embodiments disclosed herein where the filament arises vertically from the base. In certain embodiments, a cross-section of the filament in the region of the landing pad can have a planar shape to more closely match the surface contour of the wing of the catheter. For example, in certain embodiments that include two filaments, the two filaments provide landing pads for both wings of the catheter. The filaments can extend through the openings in the wings at which point the filaments loop back in the lateral direction away from the center of the catheter and are secured within the corresponding receptacle(s) in the base. In this way, the cooperative interaction between the filament(s) and the openings on the catheter and/or catheter fitting secures the catheter to the anchoring system.

In certain embodiments, the filaments extend from the base but do not rest on the base. For example, the filament can run suspended above the base which allows the filament to engage the wing even if the wing is somewhat elevated above the base without pulling the wing down in the transversal direction. In other embodiments, the filament rests on the base.

In certain embodiments, the filaments arise from the base or the filaments arise in a horizontal direction from a vertical member that is attached to the anchor pad either directly or by means of communication with the base.

In certain embodiments, the base of the anchoring system further includes an adhesive platform disposed between the filaments and facing in an upward direction to contact a portion of the catheter. Of course, the anchoring system need not include the adhesive platform in certain embodiments and can instead rely solely on the at least one filament to secure the catheter.

In one form, the present anchoring system also is adapted to receive at least several different types or styles of catheters and/or catheter fittings. In particular, the spacing between the filaments can be varied in order to accommodate catheters and/or catheter fittings with differing spacings between the suture holes. This feature can also be used to accommodate catheters and/or catheter fittings having different sizes and/or shapes. In one of the embodiments described below, the retainer includes two filaments, each of which is flexible with respect to the base of the retainer which further can accommodate variations in catheters and/or fittings. Various other aspects of the present invention, however, can be used apart from this "universal" feature, as will be apparent from the discussion of the embodiments below.

To assist in the description of these components of the anchoring system 10, the following coordinate terms are used. A "longitudinal axis" is generally parallel to the section of the catheter 12 retained by the anchoring system 10. A "lateral axis" is normal to the longitudinal axis and is generally parallel to the plane of the anchor pad 14. A "transverse axis" extends normal to both the longitudinal and lateral axes. In addition, as used herein, "the longitudinal direction" refers to a direction substantially parallel to the longitudinal axis; "the lateral direction" refers to a direction substantially parallel to the lateral axis; and "the transverse direction" refers to a direction substantially parallel to the transverse axis. Also, the terms "proximal" and "distal," which are used to describe the present anchoring system 10, are used consistently with the description of the exemplary applications. Thus, proximal and distal are used in reference to the center of the patient's body. The terms "upper," "lower," "top," "bottom," and the like, which also are used to describe the present anchoring system 10, are used in reference to the illustrations of the embodiments. A detailed description of the anchoring system 10, and its associated method of use, now follows.

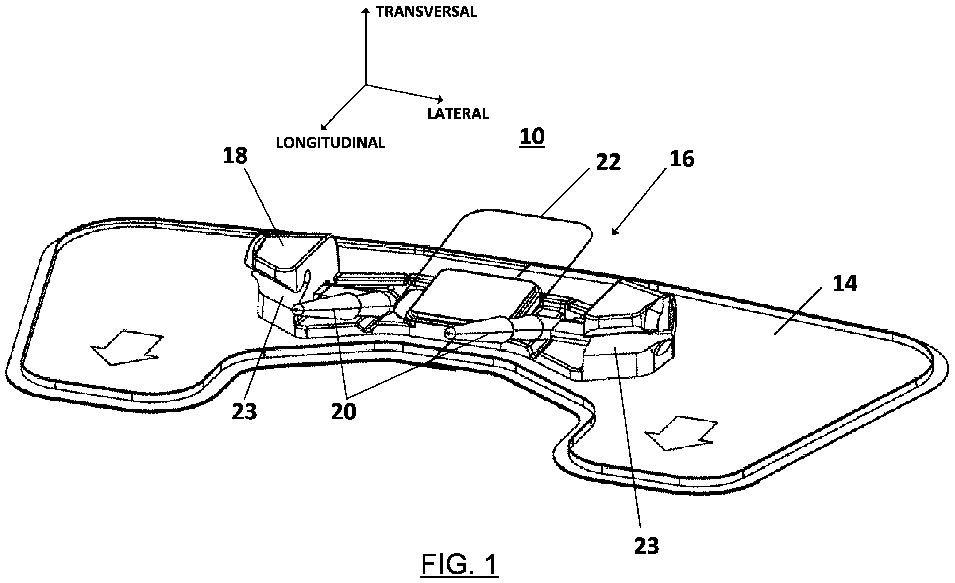

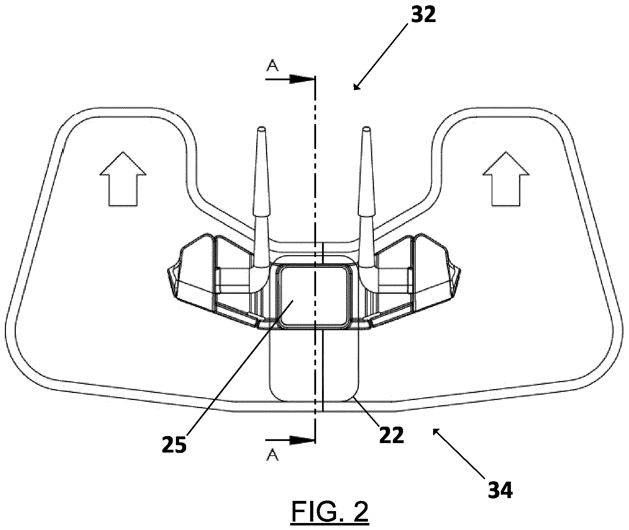

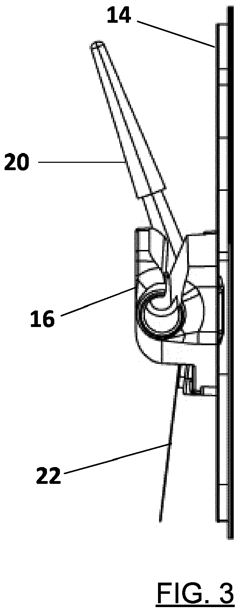

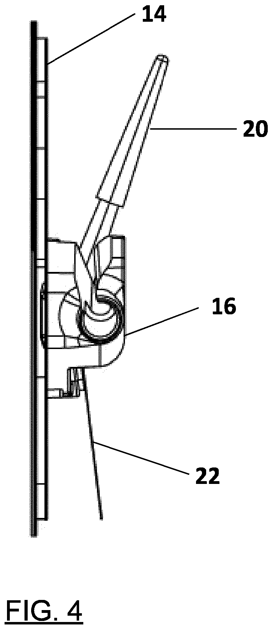

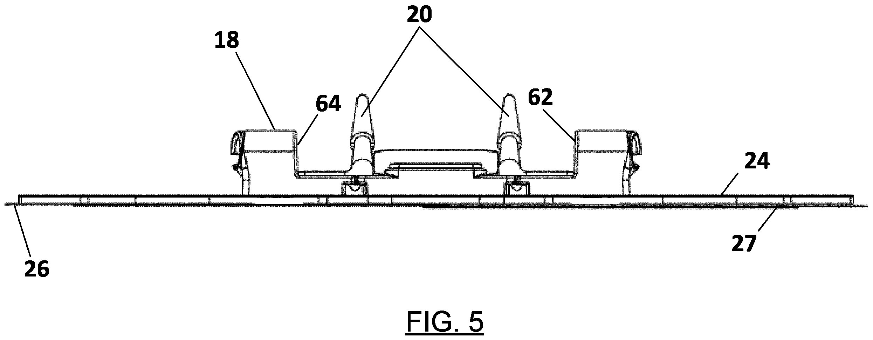

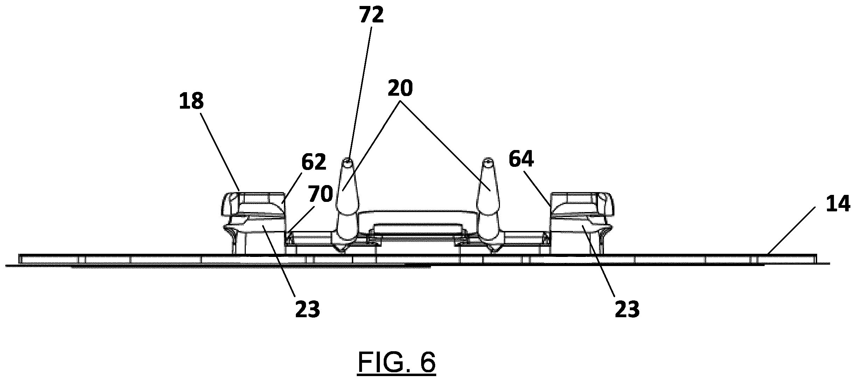

FIG. 1 is a perspective view of an anchoring system 10 in accordance with a preferred embodiment of the present invention. The anchoring system 10 comprises at least one filament 20. FIG. 2 is a top plan view of the anchoring system 10 from FIG. 1. FIG. 3 is a left side view of the anchoring system 10 from FIG. 2. FIG. 4 is a right side view of the anchoring system 10 from FIG. 2. FIG. 5 is a back view of the anchoring system 10 from FIG. 2. FIG. 6 is a front view of the anchoring system 10 from FIG. 2.

With reference to FIGS. 1-6, the anchoring system 10 is constructed in accordance with a preferred embodiment of the present invention. In certain embodiments, the anchoring system 10 comprises an anchor pad 14 and a retainer 16. The retainer 16 can include a base 18, at least one filament 20, and at least one receptacle 23. In the illustrated embodiment, each filament 20 is part of a platform 21 that is attached to the base 18 when the retainer 16 is assembled. The filament 20, as noted below, can be part of the platform 21 in certain embodiments. The filament 20 and the platform 21 can be a unitary structure in certain embodiments. The platform 21 can be part of the base 18 and/or a separate individual piece assembled to the base 18 in certain embodiments.

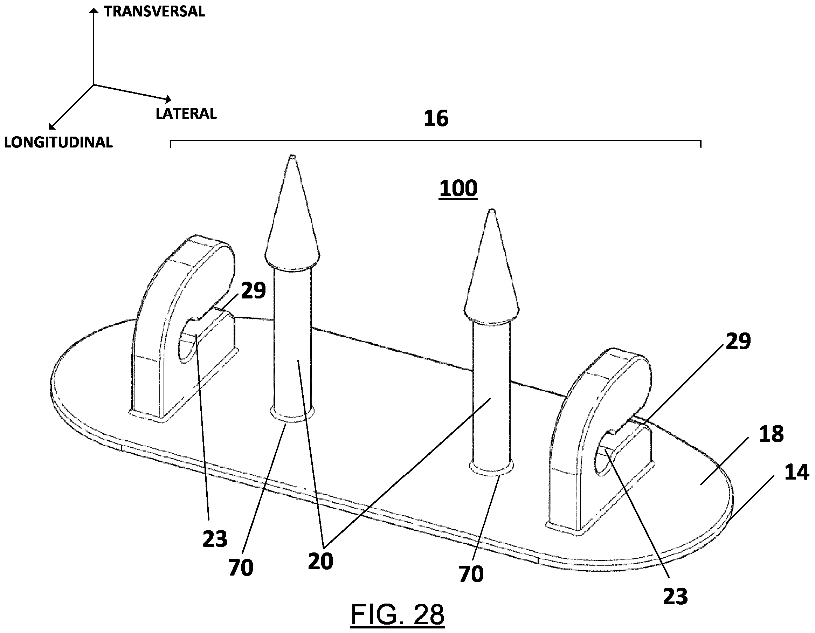

In certain embodiments, the at least one filament 20 extends in the lateral direction from a sidewall 62, 64 of the retainer 16. For example, in certain embodiments, the sidewall 62, 64 is a vertical member or surface of the base 18 that extends in the transverse direction. For example, in certain embodiments, the at least one filament 20 emerges horizontally from the vertical member or surface (e.g., sidewall 62, 64) of the base 18. Such an embodiment is in contrast to embodiments disclosed herein (e.g., FIGS. 28-36) that illustrate the at least one filament 20 arising vertically from the base 18.

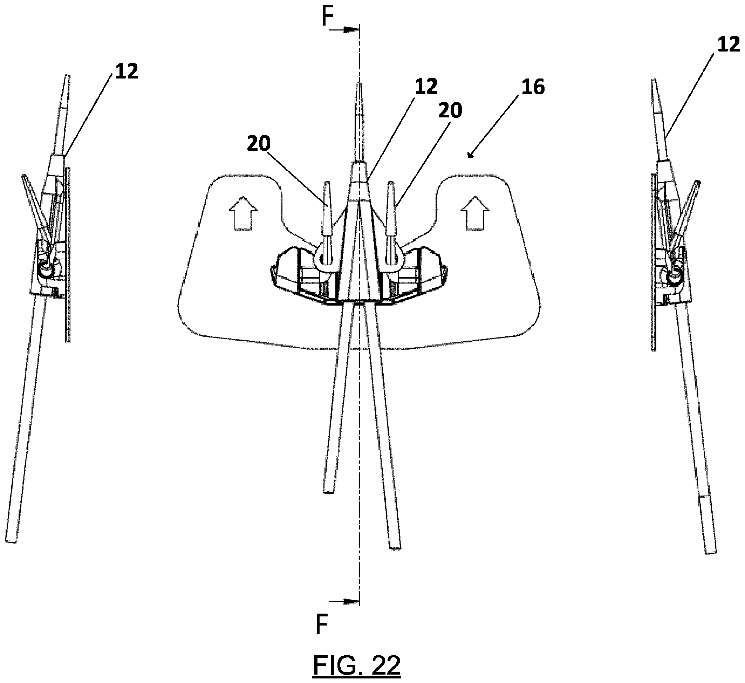

The retainer 16 is configured to accept, retain, and secure a section of a catheter 12 (shown in FIGs. 22-26) within the anchoring system 10. Alternatively, the same arrangement can be used to secure a detachable catheter fitting which is attached to a catheter 12 to be retained.

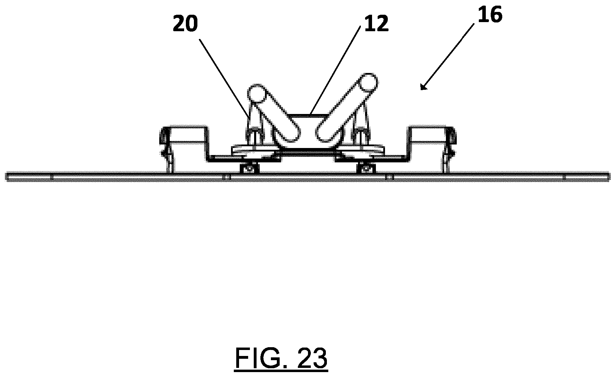

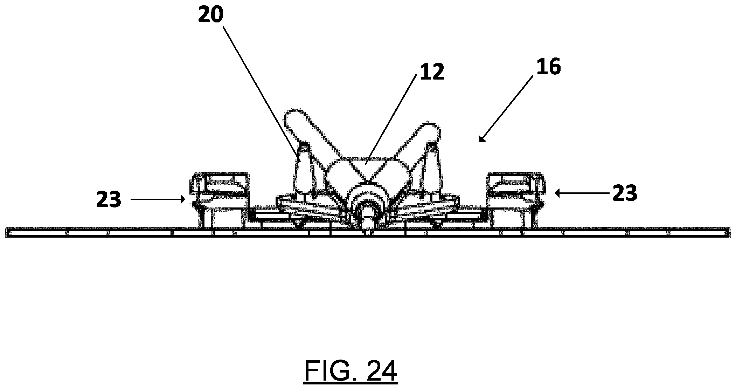

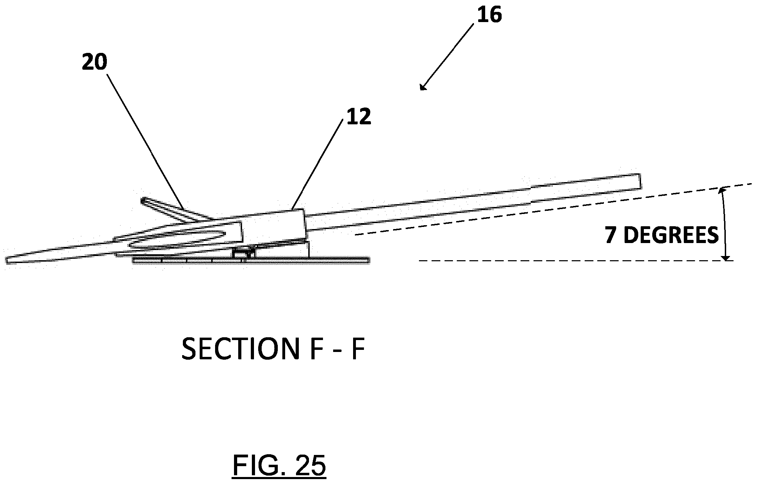

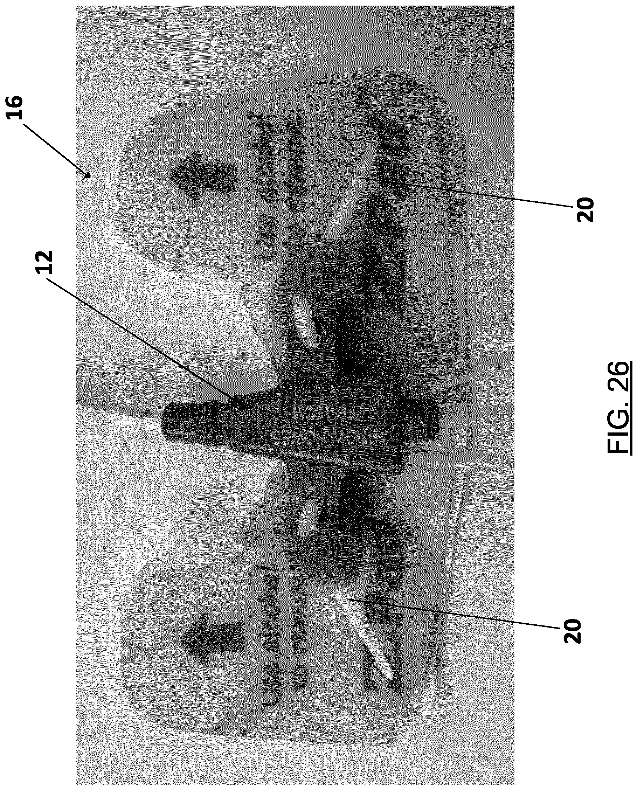

FIG. 22 includes views of a central venous catheter (CVC) 12 partially secured to the anchoring system 10 but before the at least one filament 20 is locked to the base 18. FIG. 23 is a back view of the CVC 12 and the anchoring system 10 from FIG. 22. FIG. 24 is a front view of the CVC 12 and the anchoring system 10 from FIG. 22. FIG. 25 is a cross sectional view of the CVC 12 and the anchoring system 10 from FIG. 22 taken along line F-F. FIG. 26 is a top plan view of the CVC 12 and the anchoring system 10 from FIG. 22 after the at least one filament 20 is locked to the base 18.

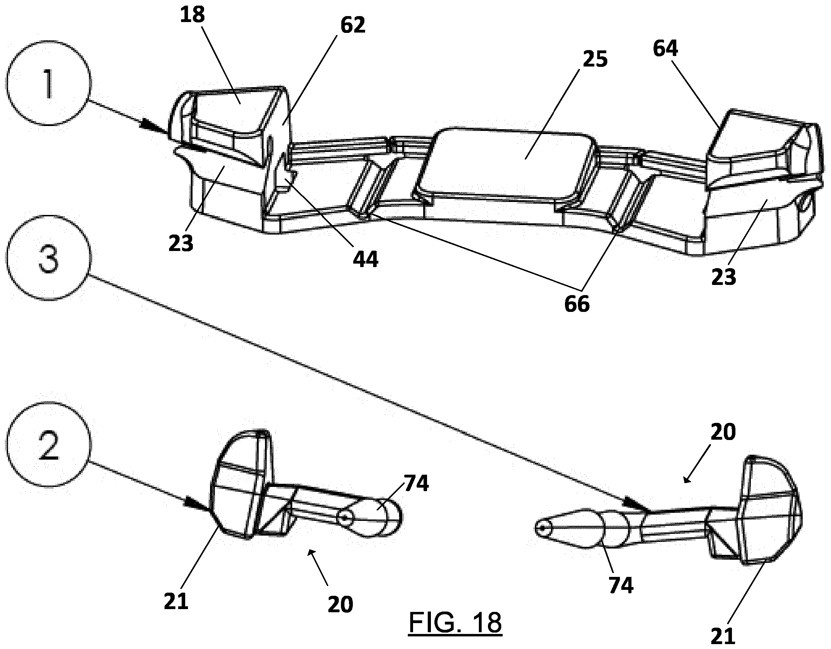

As illustrated in FIGS. 22-26, the catheter 12 or fitting is inserted into the base 18 of the retainer 16 as shown in FIG. 22. As illustrated, the at least one filament 20 in the present embodiment comprises two separate filaments 20, each independently attached to the base 18 via a platform 21 (FIG. 18). The platform 21 can be engage or assembled with the base 18 during assembly.

As the catheter 12 or fitting is positioned on the base 18, the at least one filament 20 is threaded through holes in the wings of the catheter 12 to inhibit transverse motion of the catheter 12 in the upward direction. This can be seen in FIG. 22. The at least one filament 20 is then wrapped around the wing of the catheter 12 and pulled in the lateral direction back towards the base 18 to be secured in the receptacle 23 so as to retain the catheter 12 or fitting laterally and longitudinally. The interaction of the catheter 12 with the at least one filament 20 of the retainer 16 and by its interaction with the surrounding sections of the base 18 retains the catheter 12 or fitting laterally and longitudinally. In certain embodiments that include the adhesive platform 25, the interaction of the catheter 12 with the adhesive platform 25 of the retainer 16 can further secure the fitting laterally, longitudinally, and transversely.

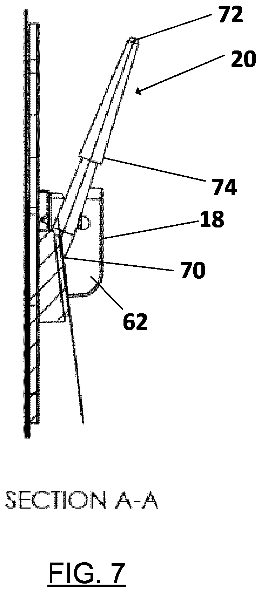

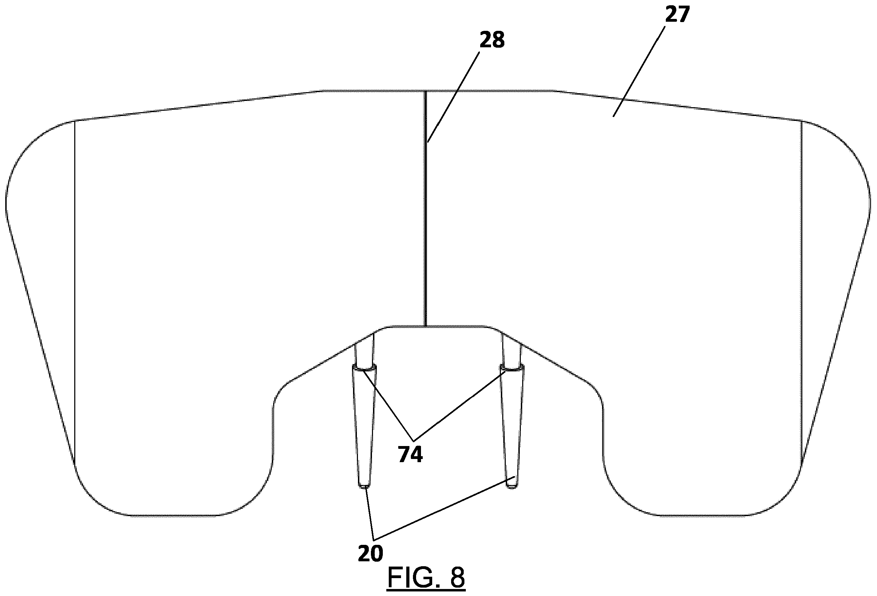

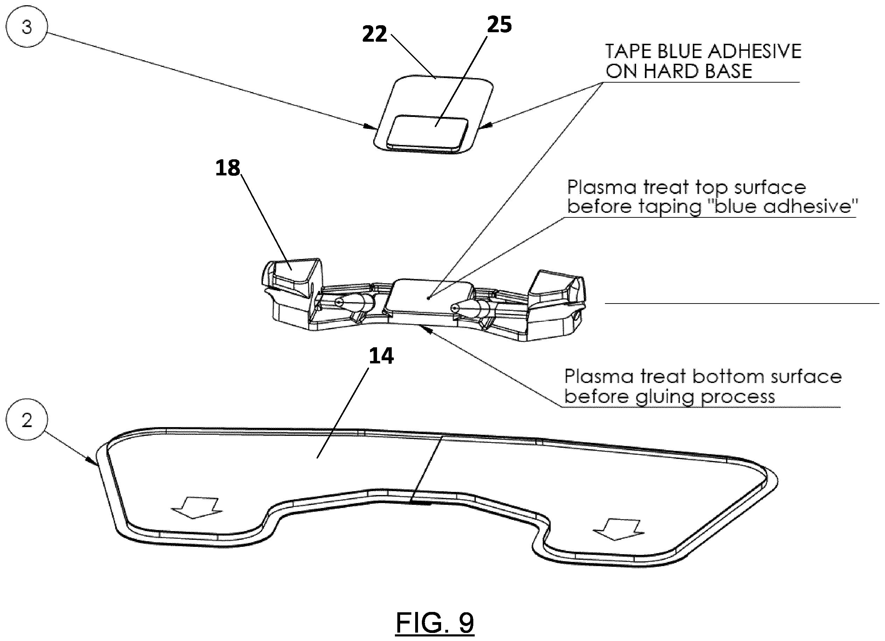

FIG. 7 is a cross sectional view of the anchoring system 10 of FIG. 2 taken along line A-A. FIG. 8 is a bottom view of the anchoring system 10 from FIG. 2. FIG. 9 is an exploded view of the anchoring system 10 from FIG. 1. The retainer 16 is disposed upon an upper surface of the anchor pad 14. The lower side 27 of the anchor pad 14 includes an adhesive surface which adheres to the skin of the patient in order to maintain the position of the retainer 16, and hence the catheter 12, with respect to the patient.

As is seen in FIG. 5, the anchor pad 14 is a substantially flat piece of material with transversely opposing sides. The proximal or lower side 27 of the anchor pad 14 faces toward the skin of the patient, and is preferably covered with an adhesive surface suitable for attaching the anchor pad 14 to the skin of the patient. The upper or distal side 24 of the pad faces away from the skin of the patient and supports the retainer 16.

The anchor pad 14 preferably comprises a laminate structure with an upper foam layer (e.g., closed-cell polyethylene foam) and a lower adhesive layer. The lower adhesive layer constitutes the lower surface 27 of the anchor pad 14. The lower surface 27 desirably is a medical-grade adhesive and can be either diaphoretic or nondiaphoretic, depending upon the particular application.

A surface of the upper foam layer constitutes the upper surface 24 of the anchor pad 14. The upper surface 24 can be roughened by chemical priming or corona-treating the foam with a low electric charge. The roughened or porous upper surface 24 can improve the quality of the adhesive joint (which is described below) between the base 18 and the anchor pad 14.

A removable paper or plastic release liner 26 desirably covers the adhesive lower surface before use. The liner 26 preferably resists tearing and desirably is divided into a plurality of pieces to ease attachment of the pad to a patient's skin. In the illustrated embodiment, the liner is split along a centerline 28 of the flexible anchor pad 14 in order to expose only half of the adhesive lower surface at one time.

The length of each liner piece, as measured in the lateral direction, extends beyond the centerline 28 of the anchor pad 14 and is folded over, or back onto the liner. This folded over portion defines a pull-tab to facilitate removal of the liner piece 26 from the adhesive lower surface. A healthcare worker uses the pull-tab by grasping and pulling on it so that the liner piece 26 is separated from the lower surface. The pull-tab eliminates the need to pick at a corner edge or other segment of the liner in order to separate the liner from the adhesive layer.

In certain embodiments, the anchor pad 14 includes a concave section 32 that narrows the center of the anchor pad 14 proximate to the retainer 16. In the illustrated embodiment of FIG. 2, the anchor pad 14 is formed generally into a crescent shape that includes a concave section 32 on one side of the retainer and a convex section 34 on the other. This shape permits the pad 14 to be placed on the patient such that the arms of the crescent extend away from the insertion site.

In certain embodiments, the retainer 16 is centered upon the anchor pad 14 about an axis which bifurcates the crescent shape. Consequently the lateral sides of the anchor pad 14 have more contact area, both forward and rearward of the retainer 16 in the longitudinal direction, which provides greater stability and adhesion to a patient's skin while still permitting the retainer 16 to be located near the insertion site.

As is illustrated in FIG. 9, in certain embodiments, the base 18 of the anchoring system 10 further includes an adhesive platform 25 attached to the base 18. In certain embodiments, the adhesive platform 25 is disposed between the at least one filament 20 with a layer of adhesive facing in an upward direction to contact and adhere to a portion of the catheter 12. The layer of adhesive can comprise a blue adhesive or any other medical grade adhesive. In certain embodiments, a top surface of the base 18 is plasma treated before taping the blue adhesive thereon. In certain embodiments, a removable paper or plastic release liner 22 covers a top surface of the layer of adhesive and can be removed prior to placing the catheter 12 in contact with the adhesive layer of the adhesive platform 25. The release liner 22 can cover the layer of adhesive before use. In the illustrated embodiment, the release liner 22 is a single piece. In other embodiments, the release liner 22 is divided into a plurality of pieces. Of course, the anchoring system 10 need not include the adhesive platform 25 in certain embodiments and can instead rely solely on the at least one filament 20 to secure the catheter 12.

In certain embodiments, a bottom surface of the base 18 is plasma treated before the base 18 is glued to the anchor pad 14.

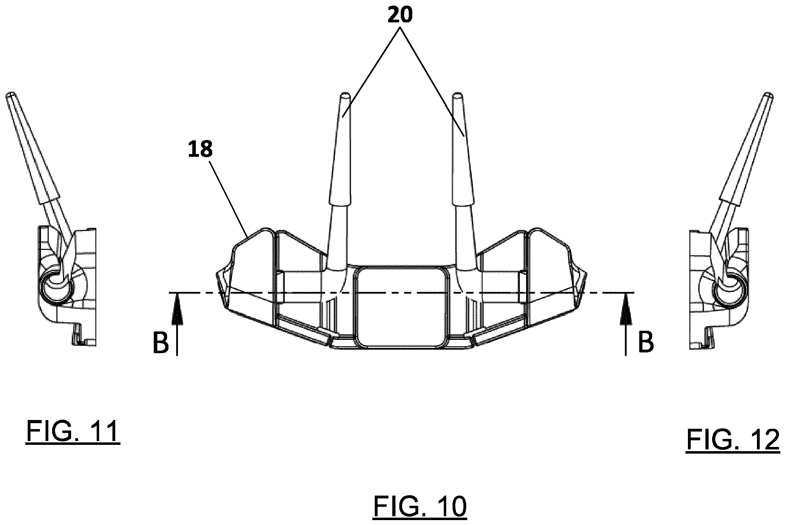

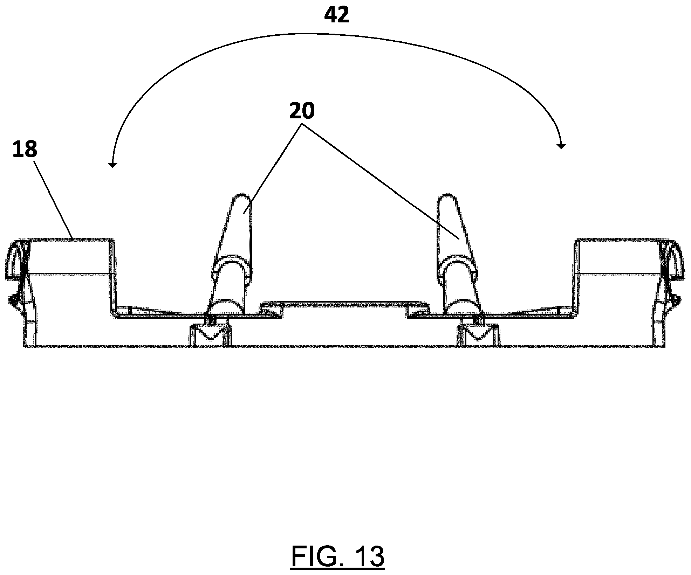

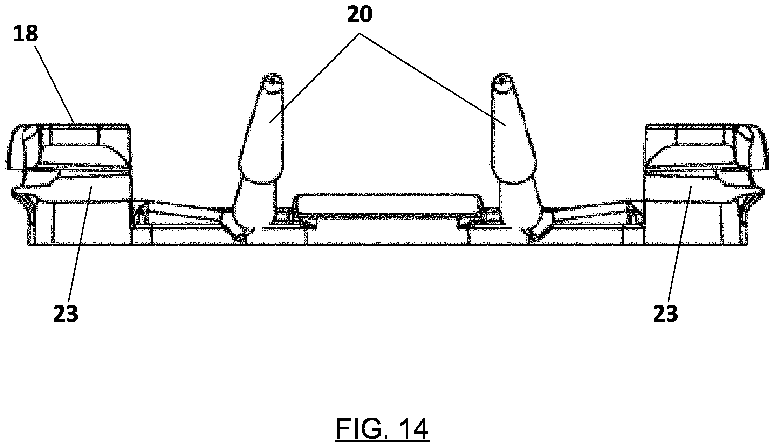

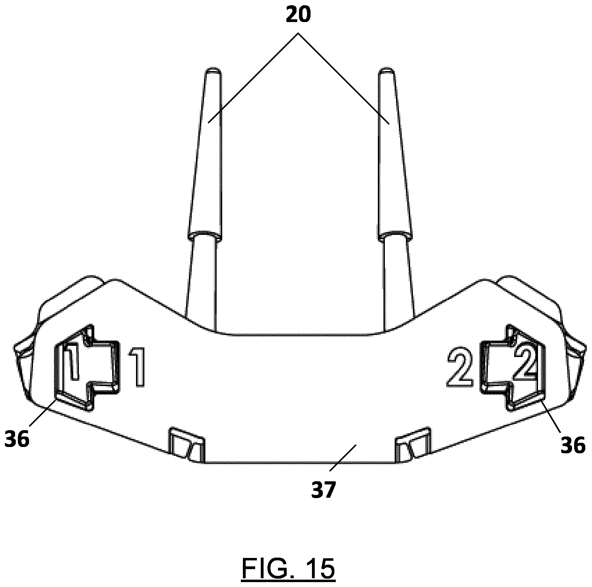

FIG. 10 is a top plan view of the retainer 16 of the anchoring system 10 from FIG. 1 with the at least one filament 20 assembled to the base 18. FIG. 11 is a left side view of the retainer 16 from FIG. 10. FIG. 12 is a right side view of the retainer 16 from FIG. 10. FIG. 13 is a back view of the retainer 16 from FIG. 10. FIG. 14 is a front view of the retainer 16 from FIG. 10. FIG. 15 is a bottom view of the retainer 16 from FIG. 10. With reference now to FIGS. 10-15, the base 18 and the at least one filament 20 principally define the retainer 16. As noted above, the at least one filament 20, in the illustrated embodiment, comprises two filaments 20, each of which is connected to the base 18 via a platform 21 during manufacture assembly. This arrangement allows the platform 21 and the filament 20 to be formed as a unitary piece prior to assembly with the base 18 to form the retainer 16.

As will become apparent, several features of the filament 20 and base 18 are desirably flexible. Suitable materials which are both sufficiently strong but flexible include without limitation: plastics, polymers, or composites such as polypropylene, polyethylene, polycarbonate, polyvinylchloride, acrylonitrile butadiene styrene, styrene butadiene, nylon, olefin, acrylic, polyester, moldable silicon, thermoplastic urethane, thermoplastic elastomers (TPE), thermoset plastics and the like. The retainer 16 can comprise a multi-piece base 18.

The base 18 can be configured in a variety of shapes, however, such as circular, square, or trapezoidal, in order to suit a particular application. For example, the base 18 may be configured to generally match the shape of the anchor pad 14 (shown in FIG. 1) or the shape of the winged catheter fitting (not shown). In the illustrated embodiment, the shape allows the base 18 to capture the somewhat rectangular shape of the catheter fitting wings (see FIG. 27).

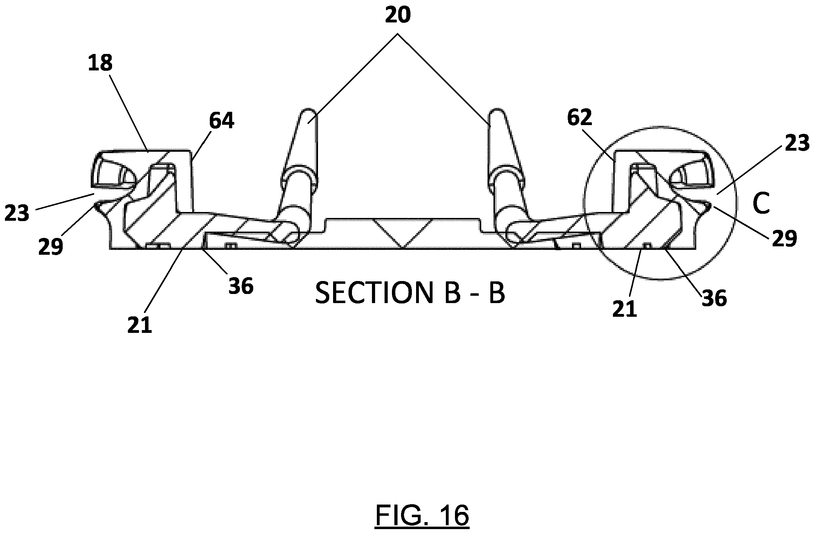

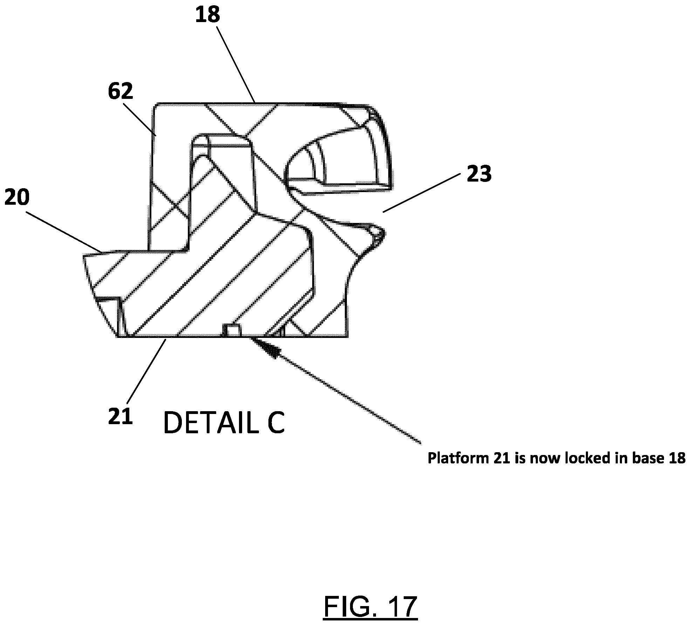

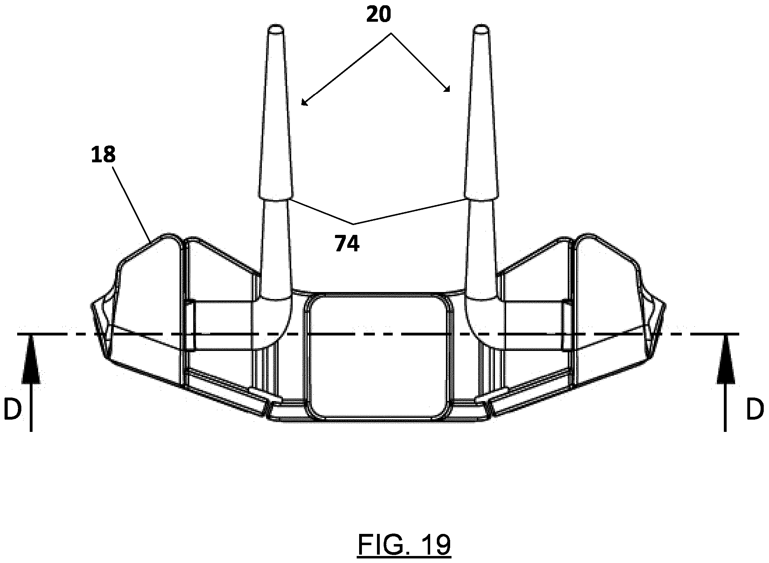

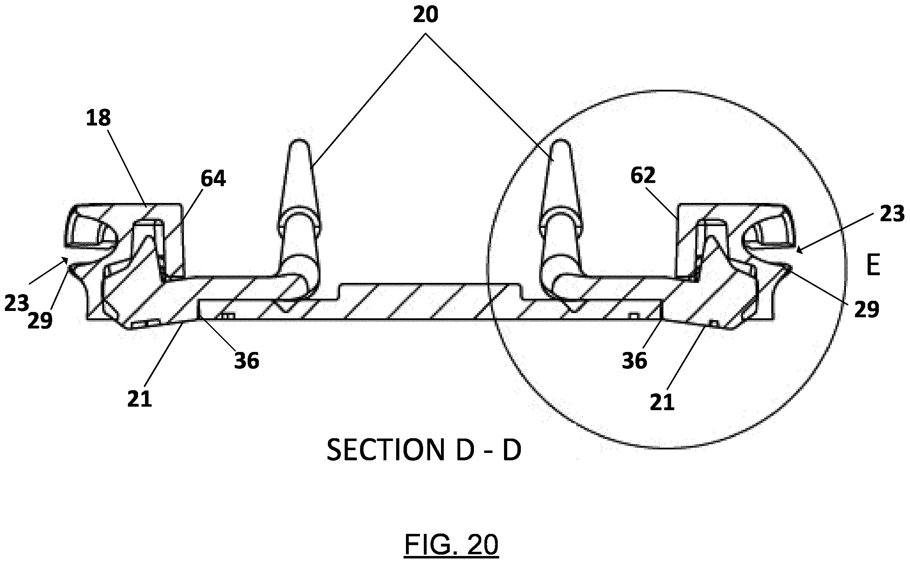

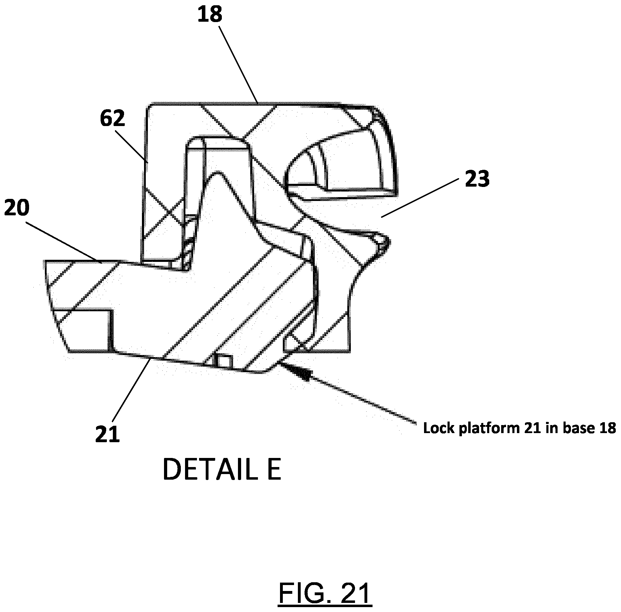

FIG. 16 is a cross sectional view of the retainer of FIG. 10 taken along line B-B showing the at least one filament 20 fully assembled to the base 18. FIG. 17 is an enlarged view from FIG. 16 taken in view C with the filament 20 pulled through the base 18 and the platform or shoulder 21 of the filament 20 locked in the base 18. FIG. 18 is an exploded view of the retainer 16 from FIG. 10 showing the at least one filament 20 prior to being assembled to the base 18. FIG. 19 is a top plan view of the retainer 16 of the anchoring system 10 from FIG. 1 with the at least one filament 20 partially assembled to the base 18. FIG. 20 is a cross sectional view of the retainer 16 of FIG. 19 taken along line D-D showing the at least one filament 20 partially assembled to the base 18. FIG. 21 is an enlarged view from FIG. 20 taken in view E with the filament 20 pulled through the base 18 but before the shoulder or platform 21 of the filament 20 is locked in the base 18.

In certain embodiments, the base 18 also includes one or more openings 36 in the base 18 to facilitate insertion of the filament 20 and associated platform or shoulder 21 so that the filament 20 passes through base 18 with the platform 21 being captured by the base 18. The one or more openings 36 can be disposed in a bottom surface 37 of the base 18. The size and shape of a distal portion of the filament 20 allows it to be inserted through the opening 36 in the bottom surface 37 and partially out the opening 44. In certain embodiments, a proximal portion of the filament 20 (e.g., platform 21) has a larger radius or size than the proximal portion of the filament 20. In certain embodiments, this proximal portion is also a larger size than the opening 44 in the base 18. Once inserted into the opening 36 so as to extend out of the opening 44, the larger size at the proximal portion of the filament (e.g., platform 21) will prevent the filament 20 from being pulled out or disengage from the base 18 in the lateral direction towards the center of the base 18.

In certain embodiments, the one or more openings 36 extend through the bottom wall 37 of the base 18 and open into a receiving space 42 defined within the retainer 16. In the illustrated embodiment, the one or more openings 36 connect to openings 44 formed in laterally facing walls of the receiving space 42; however, these openings 44 can be disposed at other locations on the base 18.

In the illustrated embodiment, the anchoring system 10 includes a pair of filaments 20 that extend in a lateral direction from the base 18. The anchoring system 10 of course can include other numbers of filaments 20 in order to suit a specific application. Each filament 20 includes a fixed proximal end 70, a free distal end 72 and at least one protuberance (generally indicated by reference numeral 74) positioned therebetween. In the illustrated embodiment, each filament 20 includes a single protuberances 74 arranged between the distal end 72 and the proximal end 74 of the filament 20.

As seen in FIG. 7, the protuberance 74 generally has a conical barb-like shape. In the illustrated embodiment, the protuberance 74 of the filament 20 has a generally conical shape with a maximum diameter at a proximal end of the protuberance 74. Although not illustrated, the protuberances 74 can take a variety of other shapes, such as for example, hollow conical shapes, arrow shapes, or transverse rib-like shapes. The proximal end of each protuberance 74, however, desirably has a diameter which is larger than the generally diameter of the filament 20.

The protuberance 74 desirably includes a needle-like shaped distal portion with a generally pointed, but blunt end portion positioned at the distal end 72 of the filament 20. In certain embodiments, the distal portion smoothly tapers with increasing diameter from the distal end 72 in a direction toward the proximal end 70.

In certain embodiments, the anchoring system 10 comprises at least one and in certain embodiments a plurality of receptacles 23 positioned on the base 18. The at least one receptacle 23 forms a channel. Each channel of the receptacle 23 is arranged on the base 18 to cooperate with at least one filament 20, as discussed below.

In certain embodiments, the channel/receptacle 23 receives a portion of the filament 20 in a manner permitting the insertion and removal of the filament 20 into a slot 29 in a side of the channel/receptacle 23, but inhibiting the retraction of the filament 20 from a front opening of the channel/receptacle 23. For this purpose, the corresponding filament 20 and receptacle 23 include interengaging structure that allows a portion of the filament 20 between the proximal and distal ends of the filament 20 to be easily inserted into and removed from the receptacle 23 via the side slot 29 in a direction (e.g., both longitudinal directions) with a first degree of force but prevents retraction of the filament 20 distal end when an even greater degree of force is applied to the filament 20 in a different direction (e.g., lateral). A much larger degree of force would be required to retract the filament 20 distal end from the receptacle 23 in the lateral direction and in many embodiments is an extreme degree of force.

In certain embodiments, the channel/receptacle 23 advantageously has a conical or funnel-like shape. In certain embodiments, a portion of the channel/receptacle 23 tapers from a large diameter to a smaller diameter. The smaller diameter desirably is smaller than the maximum diameter of the protuberances 74.

The receptacle 23 and/or the protuberance 74 are configured such that the wall along the side slot 29 of the receptacle 23 at most slightly deflects to allow the diameter of the filament 20 to pass through the side slot 29 into the receptacle 23. In the illustrated embodiment, the elastic nature of the plastic which form these components provides any required deflection necessary for the filament 20 to pass through the side slot 29. The protuberance 74 need not enter the receptacle 23 from the side slot 29 but instead is positioned outside the receptacle 23.

Once the filament 20 is stretched and passes through the side wall of the receptacle 23 with the protuberance 74 positioned beyond the end of the receptacle 23, the side wall of the receptacle 23 and the filament 20 spring back to inhibit the filament 20 from being removed from the receptacle 23 back through the side wall (e.g., longitudinal direction). The protuberance 74 is sized to prevent retraction of the filament 20 along a longitudinal axis of the receptacle 23 in the lateral direction towards the center of the retainer 16.

In certain embodiments, each filament 20 and corresponding receptacle 23 are positioned on the same side of the base 18. In the illustrated embodiment, the at least one filament 20 also is positioned on opposite sides of the base 18 from each other, and the receptacles 23 are positioned on opposite sides of the base 18 from each other.

In certain embodiments, the sidewalls 62, 64 of the base 18 are spaced laterally apart and extend upward. In certain embodiments, the at least one filament 20 extends in the lateral direction from the sidewall 62, 64 of the retainer 16. For example, in certain embodiments, the sidewall 62, 64 is a vertical member or surface of the base 18 that extends in the transverse direction. For example, in certain embodiments, the at least one filament 20 emerges horizontally from the vertical member or surface (e.g., sidewall 62, 64) of the base 18. Such an embodiment is in contrast to embodiments disclosed herein (e.g., FIGS. 28-36) that illustrate the at least one filament 20 arising vertically from the base 18.

In certain embodiments, the base 18 also includes grooves 66 which allow portions of the base 18 to flex. In certain embodiments, the longitudinal, lateral and transverse dimensions of the retainer 16 desirably are as small as possible, while still receiving and stabilizing the catheter 12, including its wings. In certain embodiments, the receiving space 42 is formed on the base 18 between the sidewalls 62, 64 of the base 18. In certain embodiments, the receiving space 42 is desirably formed so as to accept and retain a portion of the catheter 12 or catheter fitting, and in particular the wings thereof, without occluding the lumen of the catheter 12.

In operation, the self-adhesive anchor pad 14 is applied to the skin of the patient in the vicinity of the catheter 12. The anchor pad 14 should be mounted on the patient so that the at least one filament 20 and receptacles 23 are positioned on either side of the catheter and lies directly under it.

A healthcare worker places the wings of the catheter 12 on the base 18 between the at least one filament 20 and receptacles 23. In certain embodiments, the at least one filament 20 emerges horizontally from the vertical member or surface (e.g., sidewall 62, 64) of the base 18. One of the filaments 20 is threaded through the closest hole of the wing, stretched, and threaded sideways through the side slot 29 into the adjacent receptacle 23. Likewise, the healthcare worker inserts the second filament 20 through the corresponding hole of the wing, stretches the filament 20, and threads it sideways through the side slot 29 into the channel/receptacle 23. The interengaging structures of the at least one filament 20 and the corresponding receptacle 23 inhibit unintentional disengagement of the at least one filament 20 from the side slot 29 in the receptacle 23 in the longitudinal direction while preventing disengagement of the at least one filament 20 from the receptacle 23 in the lateral direction (e.g., along the longitudinal axis of the receptacle 23). The filament 20 has a sufficiently long length to wrap around the catheter 12 and easily thread through the side slot 29 into receptacle 23 with the protuberance 74 being disposed beyond the end of the receptacle 23. The healthcare worker pulls both filaments 20 tight to rigidly hold the catheter 12 between the side walls 62, 64 of the base 18. The taut filaments 20 prevent the catheter 12 from moving transversely away from the base 18 and from sliding either longitudinally or laterally over the base 18.

Excess filament 20 length is preferred and provides the healthcare worker an exposed distal end for grasping the filament 20 to then remove the filament 20 via the side slot 29 of the receptacle 23. When removal becomes necessary (e.g., removal of the catheter and/or removal of the anchoring system), the healthcare worker pulls from the distal end of the at least one filament 20 and unwraps the at least one filament 20 from the base 18. In this way, the healthcare worker can disengage the catheter 12 from the anchoring system 10 without the need for removal instruments (e.g., hemostat or scissors). The healthcare worker can simply manipulate the filaments 20 by hand to disengage the filaments 20 from the receptacles 23. Once disengaged, the healthcare worker can remove the catheter 12 from the anchoring system 10 and clean the catheter 12 if desired. Once cleaned, the healthcare worker can secure the same catheter 12 to the same anchoring system 10 by simple hand manipulation of the original filaments 20. In this way, the catheter 12 can be secured, unsecured and cleaned and secured again repeatedly without replacement or use of a removal instrument.

If desired to remove the anchoring system 10, the medical device (e.g., catheter 12) can be lifted from the base 18 after hand manipulation of the filaments 20. The anchor pad 14 can then be peeled from the patient's skin.

Thus, no painful or time-consuming sutures or other extensive procedures involving medial sharps (e.g., suture needles) are necessary to anchor a medical article to a patient's skin. Further, the medical article can be secured, unsecured and cleaned and secured again repeatedly without replacement of the anchoring system 10 or use of a removal instrument (e.g., hemostat or scissors). The anchoring system 10 allows for reversible securement. In addition, the anchor pad 14 absorbs any forces which are incurred in the installation or removal of the anchoring system 10 and the medical device, thereby providing greater comfort for the patient.

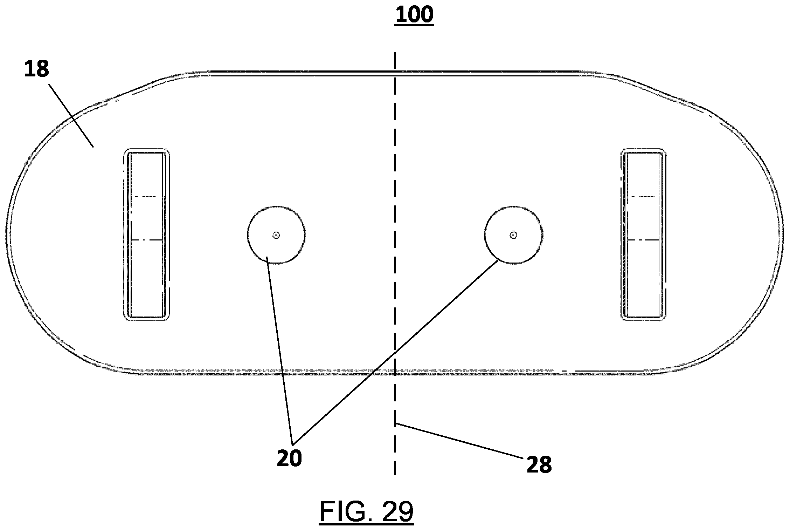

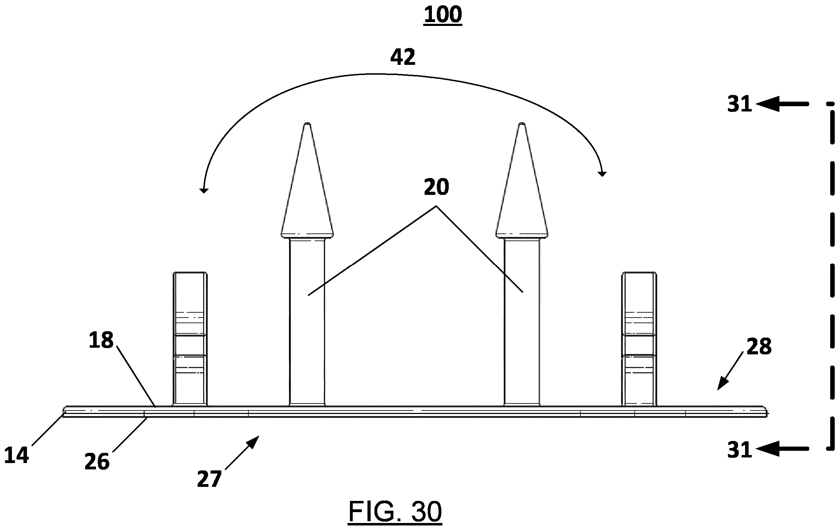

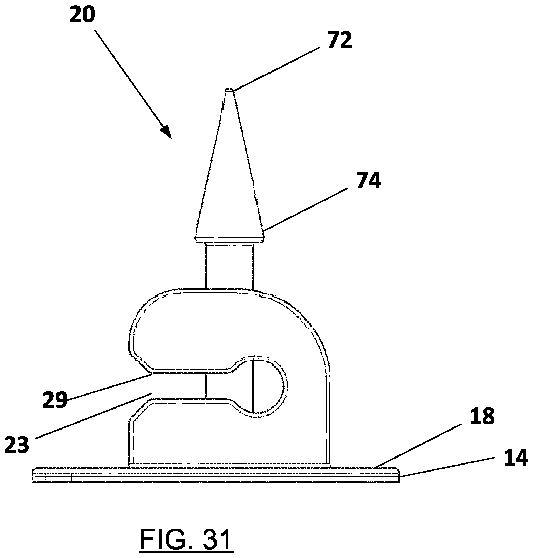

FIG. 28 is a perspective view of another preferred embodiment of an anchoring system 100 in accordance with the present invention including at least one filament 20. FIG. 29 is a top plan view of the anchoring system from FIG. 28. FIG. 30 is a front view of the anchoring system 100 from FIG. 28. FIG. 31 is a side view of the anchoring system 100 taken along lines 31-31 in FIG. 30.

With reference to FIGS. 28-31, the anchoring system 100 is constructed in accordance with a preferred embodiment of the present invention. The system comprises an anchor pad 14 and a retainer 16. The retainer 16 can include a base 18, at least one filament 20, and at least one receptacle 23. In certain embodiments, the anchor pad 14 is a substantially flat piece of material with transversely opposing sides. In certain embodiments, the proximal or lower side 27 of the pad faces toward the skin of the patient, and can be covered with an adhesive surface suitable for attaching the anchor pad 14 to the skin of the patient. The upper or distal side 24 of the pad faces away from the skin of the patient and supports the retainer 16.

In certain embodiments, a removable paper or plastic release liner 26 covers the adhesive lower surface before use. The liner 26 can resist tearing and desirably is divided into a plurality of pieces to ease attachment of the anchor pad 14 to a patient's skin. In the illustrated embodiment, the liner 26 is split along a centerline 28 of the flexible anchor pad 14 in order to expose only half of the adhesive lower surface at one time.

The length of each liner piece, as measured in the lateral direction, extends beyond the centerline 28 of the anchor pad 14 and is folded over, or back onto the liner 26. This folded over portion defines a pull-tab to facilitate removal of the liner piece 26 from the adhesive lower surface. A healthcare worker uses the pull-tab by grasping and pulling on it so that the liner piece 26 is separated from the lower surface. The pull-tab eliminates the need to pick at a corner edge or other segment of the liner in order to separate the liner 26 from the adhesive layer.

In the illustrated embodiment, the anchor pad 14 is coextensive is the longitudinal and traverse directions with the base 18. However, as illustrated in FIG. 9, the base 18 can be used with an anchor pad 14 that is not coextensive with the base 18. For example, in certain embodiments, the anchor pad 14 is larger than the base 18 and is formed generally into a crescent shape. The retainer 16 is preferably centered upon the anchor pad 14 so as to provides greater stability and adhesion to a patient's skin while still permitting the retainer 16 to be located near the insertion site.

The retainer 16 includes at least one filament 20. The filament 20 can be attached to any of the structures of the retainer 16 and extend in any direction from the structure. For example, in the embodiment illustrated in Figure 28, each filament 20 is attached to the base 18 and extends in the transversal direction. In certain other embodiments, the filament 20 is attached to the base 18 and extends in the longitudinal and/or lateral directions. In certain other embodiments, the filament 20 is attached to the side wall of the receptacle 23 and extends in the lateral direction. In certain other embodiments, the filament 20 is attached to the side wall of the receptacle 23 and extends in the longitudinal and/or transversal directions.

The retainer 16 is configured to accept and to retain and secure a section of a catheter 12 (shown in FIGs. 22-26 and 32-36) within the anchoring system 100. Alternatively, the same arrangement can be used to secure a detachable catheter fitting which is attached to a catheter 12 to be retained.

The retainer 16 is disposed upon an upper surface of the anchor pad 14. The lower side 27 of the anchor pad 14 includes an adhesive surface which adheres to the skin of the patient in order to maintain the position of the retainer 16, and hence the catheter 12, with respect to the patient.

The base 18 and the at least one filament 20 principally define the retainer 16. As noted above, the at least one filament 20, in the illustrated embodiment, comprises two filaments 20, each of which is connected to the base 18 during manufacture assembly. This arrangement allows the filament 20 to be formed as a unitary piece with the base 18 to form the retainer 16.

As will become apparent, several features of the filament 20 and base 18 are desirably flexible. Suitable materials which are both sufficiently strong but flexible include without limitation: plastics, polymers, or composites such as polypropylene, polyethylene, polycarbonate, polyvinylchloride, acrylonitrile butadiene styrene, styrene butadiene, nylon, olefin, acrylic, polyester, moldable silicon, thermoplastic urethane, thermoplastic elastomers (TPE), thermoset plastics and the like. The retainer 16 can comprise a multi-piece base 18.

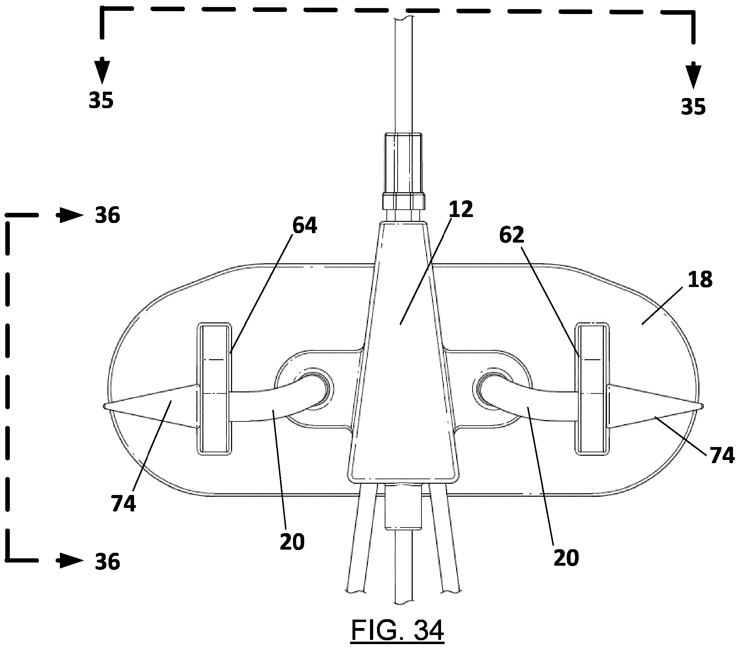

The base 18 can be configured in a variety of shapes, however, such as circular, square, or trapezoidal, in order to suit a particular application. For example, the base 18 may be configured to generally match the shape of the anchor pad 14 (shown in FIG. 28) or the shape of the winged catheter fitting (not shown). In the illustrated embodiment, the shape allows the base 18 to capture the somewhat rectangular shape of the catheter fitting wings (see FIG. 34).

In the illustrated embodiment, the anchoring system 100 includes a pair of filaments 20 that extend in a transversal direction from the base 18. Such an embodiment is in contrast to embodiments disclosed herein (e.g., FIGS. 1-26) that illustrate the filaments 20 extending in the lateral direction from a sidewall 62, 64 of the base 18.

The anchoring system 100 of course can include other numbers of filaments 20 in order to suit a specific application. Each filament 20 includes a fixed proximal end 70, a free distal end 72 and at least one protuberance (generally indicated by reference numeral 74) positioned therebetween. In the illustrated embodiment, each filament 20 includes a single protuberances 74 arranged between the distal end 72 and the proximal end 74 of the filament 20.

As seen in FIG. 31, the protuberance 74 generally has a conical barb-like shape. In the illustrated embodiment, the protuberance 74 of the filament 20 has a generally conical shape with a maximum diameter at a proximal end of the protuberance 74. Although not illustrated, the protuberances 74 can take a variety of other shapes, such as for example, hollow conical shapes, arrow shapes, or transverse rib-like shapes. The proximal end of each protuberance 74, however, desirably has a diameter which is larger than the generally diameter of the filament 20.

The protuberance 74 desirably includes a needle-like shaped distal portion with a generally pointed, but blunt end portion positioned at the distal end 72 of the filament 20. In certain embodiments, the distal portion smoothly tapers with increasing diameter from the end in a direction toward the proximal end 70.

The anchoring system 100 also includes at least one and preferably a plurality of receptacles 23 positioned on the base 18. In the embodiment illustrated in Figure 28, each receptacle 23 is disposed in a wall of the retainer 16. In the illustrated embodiment, the wall extends in an upward direction from the base 18. Each receptacle 23 is arranged on the base 18 to cooperate with at least one filament 20, as discussed below.

The channel/receptacle 23 receives a portion of the filament 20 in a manner permitting the insertion and removal of the filament 20 into a slot 29 in a side wall of the receptacle 23, but inhibiting the retraction of the filament 20 from a front opening of the receptacle 23. For this purpose, the corresponding filament 20 and receptacle 23 include interengaging structure that allows a portion of the filament 20 between the proximal and distal ends of the filament 20 to be easily inserted into and removed from the channel/receptacle 23 via the side slot 29 in a direction (e.g., both longitudinal directions) with a first degree of force but prevents retraction of the filament 20 distal end when an even greater degree of force is applied to the filament 20 in a different direction (e.g., lateral). A much larger degree of force would be required to retract the filament 20 distal end from the receptacle 23 in the lateral direction and in many embodiments is an extreme degree of force.

In certain embodiments, the channel/receptacle 23 in the lateral direction has a tubular shape. The diameter of the tubular shape desirably is smaller than a maximum diameter of the protuberances 74. In other embodiments, the channel/receptacle 23 can have a conical or funnel-like shape.

The receptacle 23 and/or the protuberance 74 are configured such that the wall along the side slot 29 of the receptacle 23 at most slightly deflects to allow the diameter of the filament 20 to pass through the side slot 29 in the longitudinal direction and into the receptacle 23. In the illustrated embodiment, the elastic nature of the plastic which forms these components provides any required deflection necessary for the filament 20 to pass through the side slot 29. The protuberance 74 need not enter the receptacle 23 from the side slot 29 but instead is positioned outside or beyond the outer lateral side of the receptacle 23.

Once the filament 20 is stretched, bent, and passes through the side wall of the receptacle 23 with the protuberance 74 positioned beyond the end of the receptacle 23, the side wall of the receptacle 23 and the filament 20 spring back to inhibit the filament 20 from being removed from the receptacle 23 back through the side wall (e.g., longitudinal direction). The protuberance 74 is sized to prevent retraction of the filament 20 along a longitudinal axis of the receptacle 23 in the lateral direction towards the center of the retainer 16.

Each filament 20 and corresponding receptacle 23 are positioned on the same side of the base 18. In the illustrated embodiment, the at least one filament 20 also is positioned on opposite sides of the base 18 from each other, and the receptacles 23 are positioned on opposite sides of the base 18 from each other.

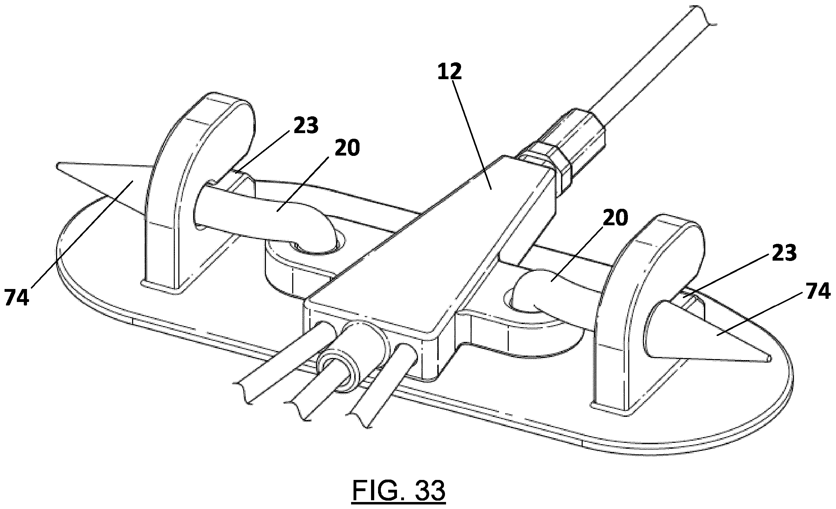

FIG. 32 is a perspective view of apertures in wings of a central venous catheter (CVC) 12 aligned for engagement with the at least one filament 20 of the anchoring system 100 of FIG. 28 during securement. FIG. 33 is similar to FIG. 32 except the at least one filament 20 has been threaded through the corresponding aperture in the CVC 12 and then fed through the corresponding receptacle 23 to lock the at least one filament 20 to the base 18 of the anchoring system 100. As is illustrated in Figure 32, the sidewalls 62, 64 of the base 18 are spaced laterally apart and extend upward. A receiving space 42 for the CVC 12 is formed on the base 18 between the sidewalls 62, 64 of the base 18. The receiving space 42 (FIG. 30) is desirably formed so as to accept and retain a portion of the CVC 12, and in particular the wings thereof, without occluding the lumen of the CVC 12.

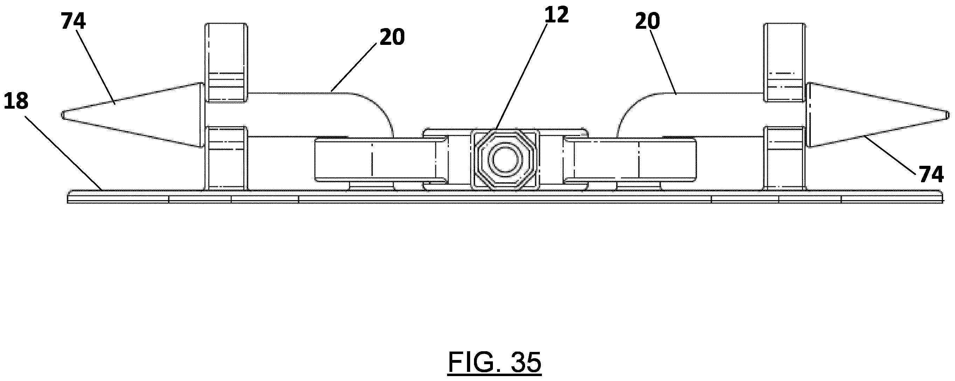

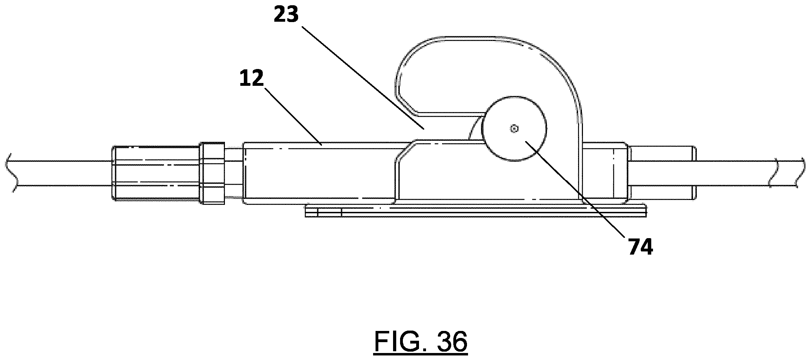

FIG. 34 is a top plan view of the CVC 12 secured to the anchoring system 100 of FIG. 28. FIG. 35 is a front view of the CVC 12 and anchoring system 100 taken along lines 35-35 in FIG. 34. FIG. 36 is a side view of the CVC 12 and anchoring system 100 taken along lines 36-36 in FIG. 34. In operation, the self-adhesive anchor pad 14 is applied to the skin of the patient in the vicinity of the catheter 12. The anchor pad 14 should be mounted on the patient so that the at least one filament 20 and receptacles 23 are positioned on either side of the catheter 12 and lie directly under it.

A healthcare worker places the wings of the catheter 12 on the base 18 between the at least one filament 20 and receptacles 23. As the catheter 12 or fitting is positioned on the base 18, the at least one filament 20 is threaded through holes in the wings of the catheter 12 to inhibit transverse motion of the catheter 12 in the upward direction.

The healthcare worker wraps each filament 20 around the wing of the catheter 12 and pull each filament 20 in the lateral direction back towards the base 18 to secure in the receptacle 23 so as to retain the catheter 12 or fitting laterally and longitudinally. The healthcare worker pulls both filaments 20 tight to rigidly hold the catheter 12 between the sidewalls 62, 64 of the base 18. The taut filaments 20 prevent the catheter 12 from moving transversely away from the base 18 and from sliding either longitudinally or laterally over the base 18.

The interengaging structures of the at least one filament 20 and the corresponding receptacles 23 inhibit unintentional disengagement of the at least one filament 20 from the side slots 29 in the receptacles 23 in the longitudinal direction while preventing disengagement of the at least one filament 20 from the receptacles 23 in the lateral direction (e.g., along the longitudinal axis of the receptacle 23).

The filament 20 has a sufficiently long length to wrap around the catheter 12 and easily thread through the side slot 29 into the receptacle 23 with the protuberance 74 being disposed beyond the end of the receptacle 23. Excess filament 20 length is preferred and provides the healthcare worker an exposed distal end for grasping the filament 20 to then remove the filament 20 via the side slot 29 of the receptacle 23. When removal becomes necessary (e.g., removal of the catheter and/or removal of the anchoring system), the healthcare worker pulls from the distal end of the at least one filament 20 and unwraps the at least one filament 20 from the base 18. In this way, the healthcare worker can disengage the catheter 12 from the anchoring system 100 without the need for removal instruments (e.g., hemostat or scissors). The healthcare worker can simply manipulate the filaments 20 by hand to disengage the filaments 20 from the receptacles 23. Once disengaged, the healthcare worker can remove the catheter 12 from the anchoring system 100 and clean the catheter 12 if desired. Once cleaned, the healthcare worker can secure the same catheter 12 to the same anchoring system 100 by simple hand manipulation of the original filaments 20. In this way, the catheter 12 can be secured, unsecured and cleaned and secured again repeatedly without replacement or use of a removal instrument.

If desired to remove the anchoring system 100, the medical device (e.g., catheter 12) can be lifted from the base 18 after hand manipulation of the filaments 20. The anchor pad 14 can then be peeled from the patient's skin.

Thus, no painful or time-consuming sutures or other extensive procedures involving medial sharps (e.g., suture needles) are necessary to anchor a medical article to a patient's skin. Further, the medical article can be secured, unsecured and cleaned and secured again repeatedly without replacement of the anchoring system 100 or use of a removal instrument (e.g., hemostat or scissors). The anchoring system 100 allows for reversible securement. In addition, the anchor pad 14 absorbs any forces which are incurred in the installation or removal of the anchoring system 100 and the medical device, thereby providing greater comfort for the patient.

Although this invention has been described in terms of a certain preferred embodiment, other embodiments apparent to those of ordinary skill in the art are also within the scope of this invention. Accordingly, the scope of the invention is intended to be defined only by the claims which follow.

Although the number of filaments 20 shown in the instant embodiment is two, any number of filaments may be used to accommodate a specific purpose. For example, if a particular catheter fitting contained four holes, a retainer designed to retain that fitting would desirably have four filaments 20.

The various embodiments of anchoring systems and techniques described above in accordance with present invention thus provide a sterile, tight-gripping, needle- and tape-free way to anchor a medical article to a patient. The retainer thus eliminates use of tape, and if prior protocol required suturing, it also reduces the risk of accidental needle sticks, suture-wound-site infections and scarring. In addition, the techniques for the described retainers can be used with any of a wide variety of catheters, fittings, tubes, wires, and other medical articles. Patient comfort is also enhanced and application time is decreased with the use of the present anchoring system.

### Terminology

Although certain embodiments and examples are disclosed herein, inventive subject matter extends beyond the examples in the specifically disclosed embodiments to other alternative embodiments and/or uses, and to modifications and equivalents thereof. Thus, the scope of the claims appended hereto is not limited by any of the particular embodiments described above. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

Furthermore, certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as a subcombination or variation of a subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

For expository purposes, the term "horizontal" as used herein is defined as a plane parallel to the plane or surface of the floor or ground of the area in which the device being described is used or the method being described is performed, regardless of its orientation. The term "floor" floor can be interchanged with the term "ground." The term "vertical" refers to a direction perpendicular to the horizontal as just defined. Terms such as "above," "below," "bottom," "top," "side," "higher," "lower," "upper," "over," and "under," are defined with respect to the horizontal plane.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without other input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

## Claims

1. An anchoring device for securing a catheter (12) to the body of a patient, the catheter (12) having a wing having at least one suture hole formed therein, the anchoring device comprising:
an adhesive member (14) for secure attachment to the body of the patient;
a base (18) mounted on the adhesive member (14) and having at least one receptacle (23), the at least one receptacle (23) forming a channel through a portion of the base, the channel having a central axis and a slot (29) through a side of the channel; and
at least one filament (20) having a proximal end, a distal end, and a midportion therebetween, the proximal end being coupled to the base (18), the distal end being configured to be inserted through the suture hole of the wing, the midportion being sized and shaped to be inserted into and subsequently removed from the channel via the slot (29),
wherein the at least one filament (20) is inhibited from being withdrawn from the channel in a direction along the central axis towards the wing when the midportion of the at least one filament (20) is in the channel.

2. The apparatus of claim 1, wherein the central axis is parallel to a lateral direction.

3. The apparatus of any one of the preceding claims, wherein the base (18) comprises at least one sidewall (62, 64), and wherein the at least one filament (20) extends in a lateral direction from the at least one sidewall (62, 64).

4. The apparatus of claim 3, wherein the at least one sidewall (62, 64) is a vertical member or surface.

5. An anchoring system (10, 100) for securing a catheter (12) to the body of a patient comprising:
a catheter (12) comprising a wing having at least one suture hole formed therein; and
an anchoring apparatus (16) comprising:
an adhesive member (14) for secure attachment to the body of the patient;
a base (18) mounted on the adhesive member (14) and having at least one receptacle (23), the at least one receptacle (23) forming a channel through a portion of the base, the channel having a central axis and a slot (29) through a side of the channel; and
at least one filament (20) having a proximal end, a distal end, and a midportion therebetween, the proximal end being coupled to the base (18), the distal end being configured to be inserted through the suture hole of the wing, the midportion being sized and shaped to be inserted into and subsequently removed from the channel via the slot (29),
wherein the at least one filament (20) is inhibited from being withdrawn from the channel in a direction along the central axis towards the wing when the midportion of the at least one filament (20) is in the channel.

6. The apparatus of any one of the preceding claims, wherein the at least one filament (20) comprises a platform (21) configured to lock to the base (18).

7. The apparatus of any one of the preceding claims, wherein the at least one filament (20) comprises a protuberance configured to abut a surface of the receptacle (23) when the midportion of the at least one filament (20) is in the channel.

8. The apparatus of any one of the preceding claims, wherein the base (18) further comprises an adhesive platform (25) configured to contact at least a portion of the catheter (12).

9. The apparatus of any one of the preceding claims, wherein the channel has a C-shape.

10. The apparatus of any one of the preceding claims, wherein the slot (29) faces in a longitudinal direction towards an insertion site.

11. The apparatus of any one of the preceding claims, wherein the at least one receptacle (23) comprises two receptacles and the at least one filament (20) comprises two filaments.

12. The apparatus of Claim 11, wherein the midportions of the two filaments extend along a lateral direction when secured in the two receptacles.

13. The apparatus of claim 12, wherein the midportions of the two filaments extend in opposite lateral directions away from each other when secured in the two receptacles.

14. The apparatus of claim 5, wherein the base (18) comprises at least one sidewall (62, 64), and wherein the at least one filament (20) extends in a lateral direction from the at least one sidewall (62, 64).

15. The apparatus of claim 14, wherein the at least one sidewall (62, 64) is a vertical member or surface.
